# EUROPÄISCHE PATENTANMELDUNG

(11) **EP 2 065 370 A1**
(43) Veröffentlichungstag der Anmeldung: **03.06.2009**
(21) Anmeldenummer: 07119747.9
(22) Anmeldetag: 31.10.2007
(51) Int. Cl.: C07D 213/42, C07D 249/12, C07D 277/20, A01N 43/04, A01N 43/24, A01N 43/34, A01N 43/48

(54) **2-Cyanobenzolsulfonamide als Pestizide**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung beinhaltet 2-Cyanobenzolsulfonamide der Formel (I) 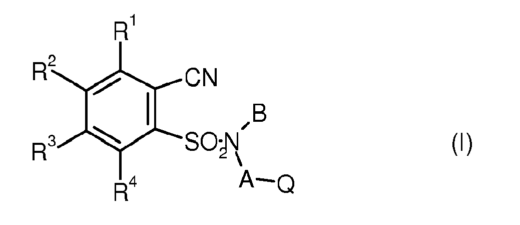 in welcher A, R¹, R², R³, R⁴ und Q und B die in der Beschreibung angegebenen Bedeutungen haben, mehrere Verfahren zu ihrer Herstellung und ihre Verwendung als Wirkstoffe, insbesondere ihre Verwendung als Schädlingsbekämpfungsmittel.

## Beschreibung

Die vorliegende Erfindung betrifft neue Schädlingsbekämpfungsmittel, mehrere Verfahren zu ihrer Herstellung und ihre Verwendung als Wirkstoffe, insbesondere ihre Verwendung als Insektizide und /oder Akarizide.

Die EP 0033984 beschreibt substituierte 2-Cyanobenzolsulfonamidverbindungen mit blattlausabtötender Wirksamkeit. Weiterhin bekannt wurden 2-Cyanobenzolsulfonamide in WO 2005/035486, WO 2006/056433 und WO 2007/060220 mit insektizider Wirkung. Deren Anwendung für Boden- und Saatgutanwendungen ist in WO2006/100271 und WO 2006/100288 beschrieben. Auf diese Veröffentlichungen wird hier mit Bezug genommen.

Es wurden nun neue 2-Cyanobenzolsulfonamide der Formel (I) gefunden 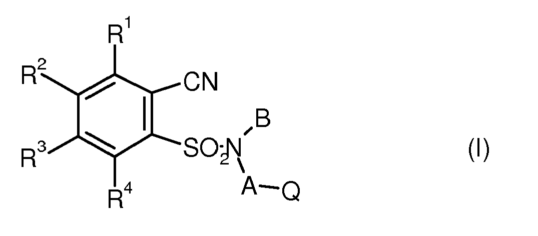 in welcher
- A: für C₁-C₆-Alkandiyl, C₃-C₆-Cycloalkyldiyl, C₂-C₆-Alkendiyl, C₁-C₆-Alkyl-C₂-C₆-Alkendiyl-, C₁-C₄-Alkyloxydiyl, C₁-C₄-Alkyloxy-C₁-C₄-alkyldiyl, C₁-C₄-Alkylthio-C₁-C₄-alkyldiyl, C₁-C₄-Alkylsulfinyl-C₁-C₄-alkyldiyl, C₁-C₄-Alkylsulfonyl-C₁-C₄-alkyldiyl steht,
wobei
diese Reste unsubstituiert sein können oder einen, zwei oder mehrere Reste aus der Gruppe Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino, Di-(C₁-C₄-alkyl)amino, Aryl oder Hetaryl tragen können;
- B: für A-Q, Cyano, C₁-C₆-Alkyl, C₃-C₈-Cycloalkyl, C₂-C₆-Alkenyl, C₁-C₆-Alkyl-C₂-C₆-Alkenyl, C₂-C₆-Alkinyl, C₁-C₄-Alkoxy, C₃-C₈-Cycloalkyl, -C(=X)-R⁵ , C₁-C₄-Alkylsulfonyl, (C₁-C₄-Alkyl)amino, Di-(C₁-C₄-alkyl)amino, Aryl, Hetaryl, C₁-C₆-Arylalkyl und C₁-C₆-Hetarylalkyl steht, wobei die vier letztgenannten Reste unsubstituiert sein können oder einen, zwei oder mehrere Reste aus der Gruppe Halogen, Cyano, C₁-C₆-Alkyl, C₃-C₈-Cycloalkyl, Phenyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können;
- B: weiterhin für den Rest 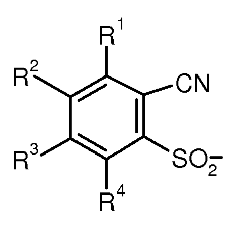
steht;
- Q: für einen 5-, 6- oder 7-gliedrigen gesättigten oder ungesättigten heterocyclischen Ring oder ein 9- oder 10-gliedriges annelliertes heterobicyclisches Ringsystem steht, wobei der Ring oder das Ringsystem, gegebenenfalls einfach oder mehrfach, gleich oder verschieden substituiert ist, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, Di-(C₁-C₄-alkyl)aminocarbonyl, (C₁-C₄-Alkyl)(C₁-C₄-Alkoxy)imino,
oder
wobei die Substituenten unabhängig voneinander ausgewählt sein können aus Phenyl oder einem 5-oder 6-gliedrigen heteroaromatischen Ring, wobei Phenyl oder der heteroaromatische Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy substituiert sein können,
- Q: weiterhin für einen der folgenden Reste Q1 bis Q111 steht 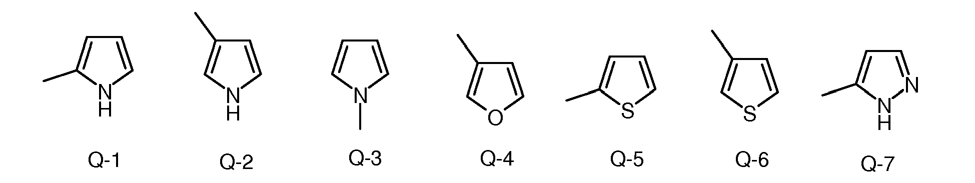 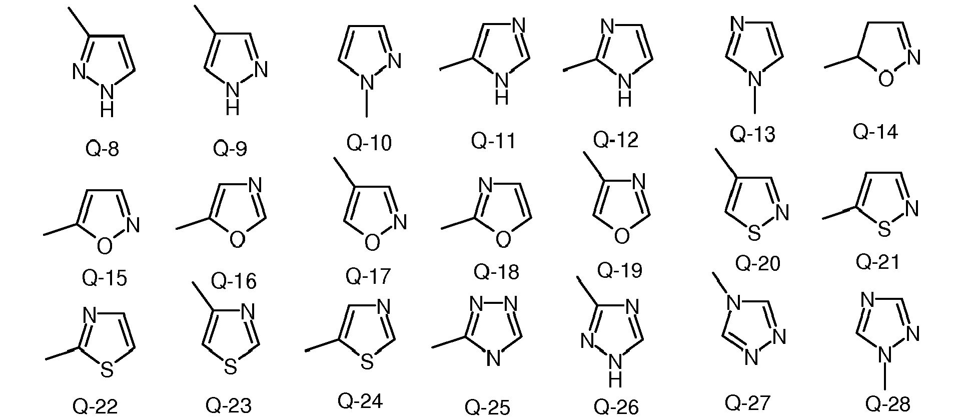 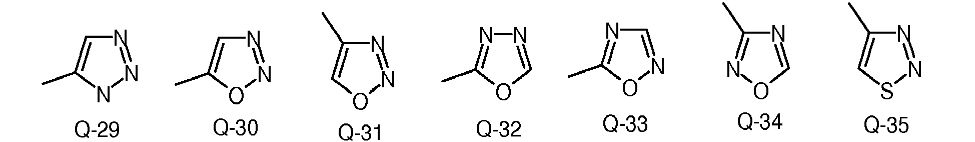 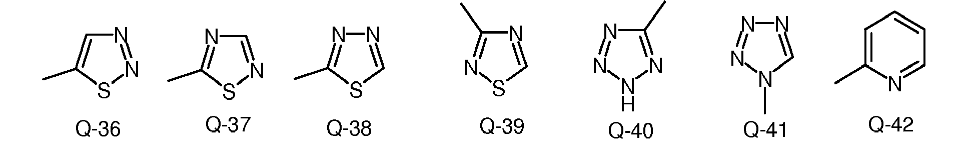 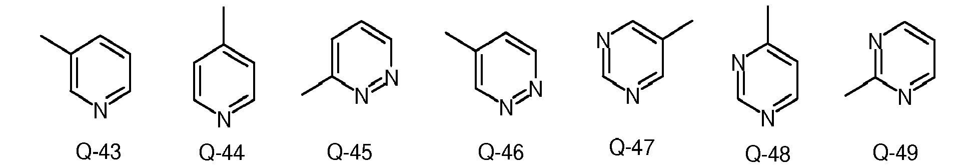 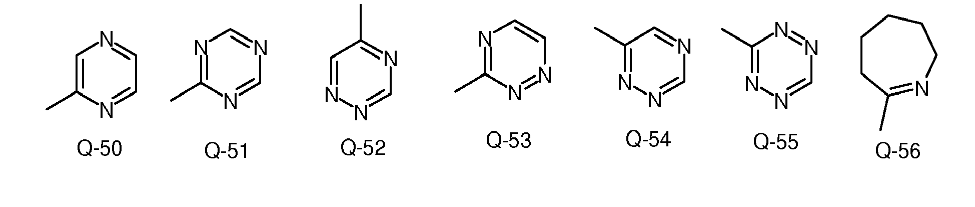 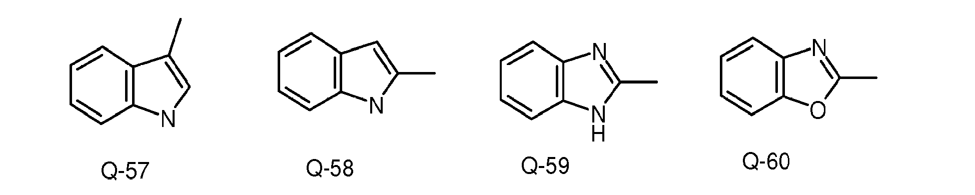 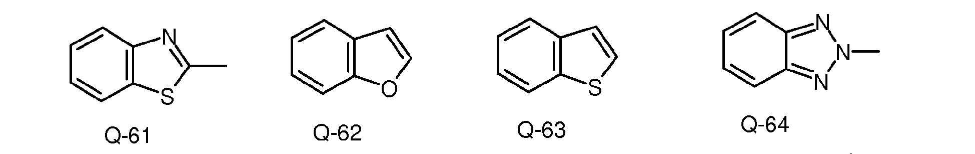 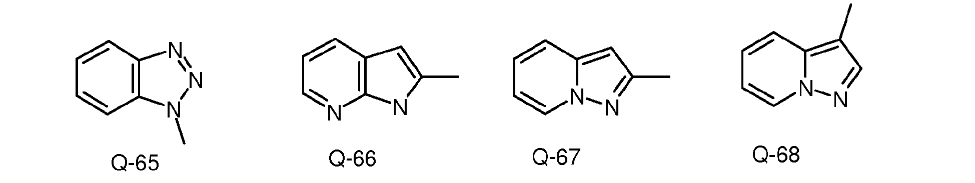 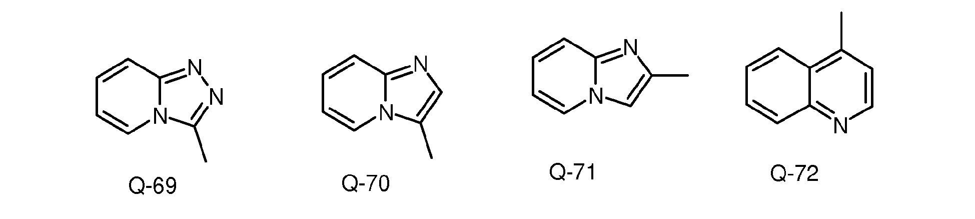 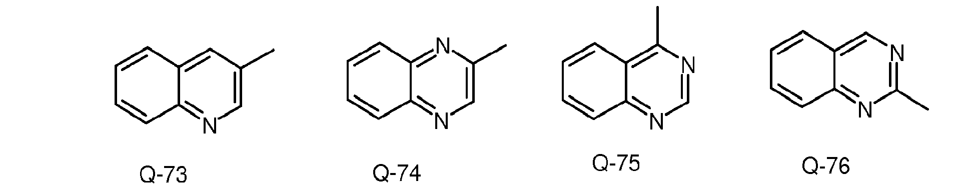 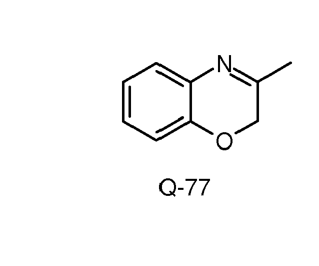 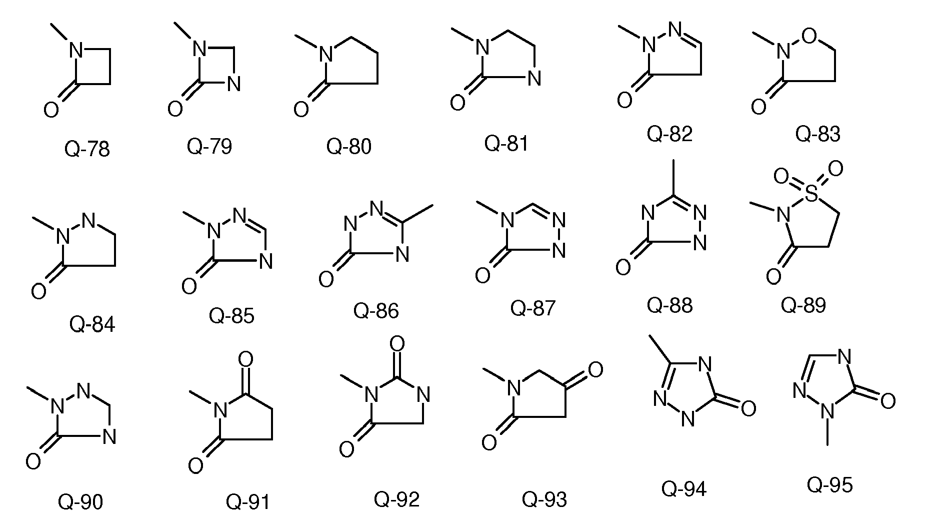 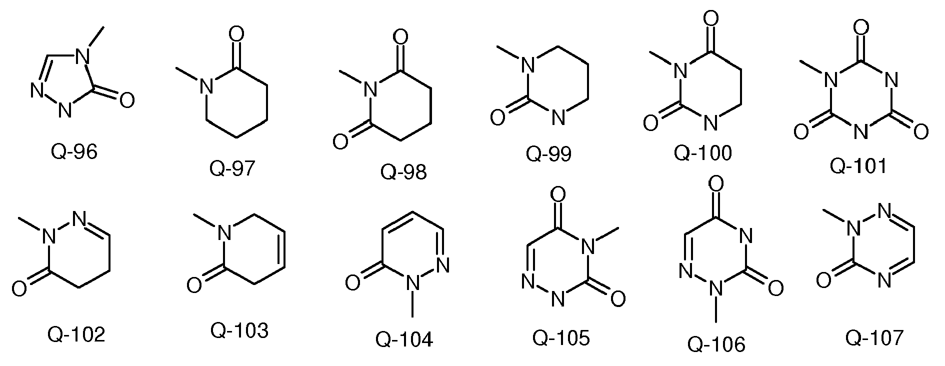 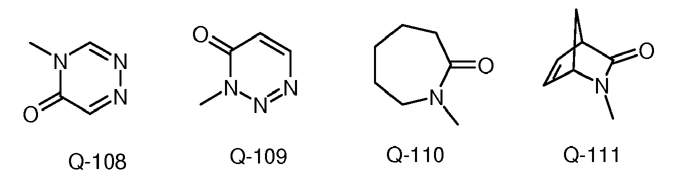
- R¹: für Wasserstoff, Halogen, C₁-C₆-Alkyl, C₁-C₆-Halogenalkyl, C₁-C₄-Alkoxy, C₃-C₆-Alkenyloxy, Methoxyethoxy oder C₁-C₄-Halogenalkoxy steht;
- R², R³ und R⁴: unabhängig voneinander für Wasserstoff, Halogen, Cyano, Nitro, C₁-C₆-Alkyl, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy, C₁-C₄-Halogenalkylthio, C₂-C₆-Alkenyl, C₂-C₆-Alkinyl, C₁-C₄-Alkoxycarbonyl, Amino, (C₁-C₄-Alkyl)amino, Di-(C₁-C₄-alkyl)amino, Aminocarbonyl, (C₁-C₄-Alkyl)aminocarbony, Di-(C₁-C₄-alkyl)aminocarbonyl, Aryl und Hetaryl stehen,
wobei
die beiden letztgenannten Reste unsubstituiert sein können oder einen, zwei oder mehrere Reste aus der Gruppe Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können;
- R⁵: für C₁-C₆-Alkyl, C₃-C₈-Cycloalkyl, C₁-C₄-Halogenalkyl, C₁-C₄-Alkoxy, C₁-C₄-Alkylthio, C₂-C₆-Alkenyl, C₂-C₆-Alkinyl, Aryl und Hetaryl steht, wobei diese Reste unsubstituiert sein können oder einen, zwei oder mehrere Reste aus der Gruppe C₁-C₆-Alkyl, C₃-C₆-Cycloalkyl, Halogen, Cyano, Nitro, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy und C₁-C₄-Halogenalkylthio, Aryl und Hetaryl tragen können, obei die beiden letztgenannten Reste unsubstituiert sein können oder einen, zwei oder mehrere Reste aus der Gruppe Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können;
- X: für O oder S steht;
die Verbindungen der allgemeinen Formel (I) außerdem N-Oxide und Salze umfassen.
Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.
Die erfindungsgemäßen 2-Cyanobenzolsulfonamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- A: steht bevorzugt für C₁-C₆-Alkandiyl, C₃-C₆-Cycloalkyldiyl, C₂-C₆-Alkendiyl, C₁-C₆-Alkyl-C₂-C₆-Alkendiyl-, C₁-C₄-Alkyloxydiyl, C₁-C₄-Alkyloxy-C₁-C₄-alkyldiyl, C₁-C₄-Alkylthio-C₁-C₄-alkyldiyl, C₁-C₄-Alkylsulfinyl-C₁-C₄-alkyldiyl und C₁-C₄-Alkylsulfonyl-C₁-C₄-alkyldiyl;wobei diese Reste unsubstituiert sein können oder einen, zwei oder mehrere Reste aus der Gruppe Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino, Di-(C₁-C₄-alkyl)amino, Aryl oder Hetaryl tragen können;
- A: steht besonders bevorzugt für C₁-C₆-Alkandiyl, C₃-C₆-Cycloalkyldiyl, C₂-C₆-Alkendiyl, C₁-C₆-Alkyl-C₂-C₆-Alkendiyl-, C₁-C₄-Alkyloxydiyl, C₁-C₄-Alkyloxy-C₁-C₄-alkyldiyl, wobei diese Reste unsubstituiert sein können oder einen, zwei oder mehrere Reste aus der Gruppe Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio tragen können;
- A: steht ganz besonders bevorzugt für Methylen, Ethylen
- B: steht bevorzugt für A-Q, Cyano, C₁-C₆-Alkyl, C₃-C₈-Cycloalkyl, C₂-C₆-Alkenyl, C₁-C₆-Alkyl-C₂-C₆-Alkenyl, C₂-C₆-Alkinyl, C₁-C₄-Alkoxy, C₃-C₈-Cycloalkyl, -C(=X)-R⁵, C₁-C₄-Alkylsulfonyl, (C₁-C₄-Alkyl)amino, Di-(C₁-C₄-alkyl)amino, Aryl, Hetaryl, C₁-C₆-Arylalkyl und C₁-C₆-Hetarylalkyl, wobei die vier letztgenannten Reste unsubstituiert sein können oder einen, zwei oder mehrere Reste aus der Gruppe Halogen, Cyano, C₁-C₆-Alkyl, C₃-C₈-Cycloalkyl, Phenyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können..
- B: steht weiterhin bevorzugt für 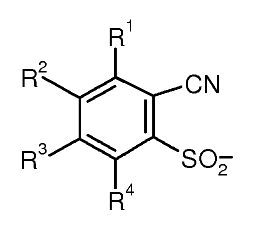
- B: steht besonders bevorzugt für A-Q, Cyano, C₁-C₆-Alkyl, C₃-C₈-Cycloalkyl, C₂-C₆-Alkenyl, C₁-C₆-Alkyl-C₂-C₆-Alkenyl, C₂-C₆-Alkinyl, C₁-C₄-Alkoxy, -C(=O)-R⁵, C₁-C₄-Alkylsulfonyl, Aryl, Hetaryl, C₁-C₆-Arylalkyl und C₁-C₆-Hetarylalkyl, wobei die vier letztgenannten Reste unsubstituiert sein können oder einen, zwei oder mehrere Reste aus der Gruppe Halogen, Cyano, C₁-C₆-Alkyl, C₃-C₈-Cycloalkyl, Phenyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy und C₁-C₄-Halogenalkylthio tragen können,
- B: steht weiterhin besonders bevorzugt für 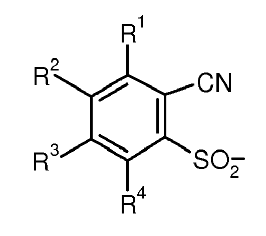
- B: steht ganz besonders bevorzugt für A-Q, C₁-C₆-Alkyl, Acetyl und 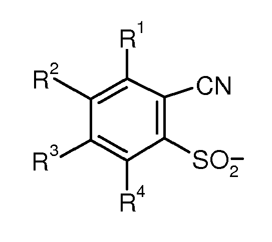
- R¹: steht bevorzugt für Wasserstoff, Halogen, C₁-C₆-Alkyl, C₁-C₆-Halogenalkyl, C₁-C₄-Alkoxy
- R¹: steht besonders bevorzugt für Wasserstoff, Halogen, C₁-C₃-Alkyl, C₁-C₃-Halogenalkyl, C₁-C₃-Alkoxy
- R¹: steht ganz besonders bevorzugt für Methoxy, Ethoxy, Fluor, Chlor , Brom, Methyl, Ethyl, Trifluormethyl, OCHF₂, OCF₃ , OCClF₂
- R², R³ und R⁴: steht bevorzugt für Wasserstoff, Halogen, Cyano, Nitro, C₁-C₆-Alkyl, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy, C₁-C₄-Halogenalkylthio, C₂-C₆-Alkenyl, C₂-C₆-Alkinyl, C₁-C₄-Alkoxycarbonyl, (C₁-C₄-Alkyl)amino, Di-(C₁-C₄-alkyl)amino
- R², R³ und R⁴: steht besonders bevorzugt für Wasserstoff, Halogen, Cyano, C₁-C₂-Alkoxy , C₁-C₃-Alkyl, C₁-C₃-Haloalkyl , C₁-C₂-Halogenalkoxy oder Di-(C₁-C₂-alkyl)amino
- R², R³ und R⁴: steht ganz besonders bevorzugt für Wasserstoff, Methyl, Ethyl, Fluor, Chlor, Brom, Jod, Trifluormethyl, Methoxy, Ethoxy, Dimethylamino
- Q: steht bevorzugt für einen gegebenenfalls einfach oder mehrfach substituierten 5-, 6- oder 7-gliedrigen gesättigten oder ungesättigten heterocyclischen Ring oder ein 9- oder 10-gliedriges annelliertes heterobicyclisches Ringsystem der Reihe Q-1 bis Q-111, wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, Di-(C₁-C₄-alkyl)aminocarbonyl, (C₁-C₄-Alkyl)(C₁-C₄-Alkoxy)imino,
oder wobei die Substituenten unabhängig voneinander ausgewählt sein können aus Phenyl oder einem 5- oder 6-gliedrigen heteroaromatischen Ring, wobei Phenyl oder der heteroaromatische Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy substituiert sein können,
- Q: steht besonders bevorzugt für einen gegebenenfalls einfach oder mehrfach substituierten 5-, 6- oder 7-gliedrigen gesättigten oder ungesättigten heterocyclischen Ring oder ein 9-oder 10-gliedriges annelliertes heterobicyclisches Ringsystem der Reihe Q-1 bis Q-55 und Q-60 bis Q-111 ,wobei die Substituenten unabhängig voneinander ausgewählt sein können aus C₁-C₃-Alkyl, C₂-C₃-Alkenyl, C₂-C₃-Alkinyl, C₃-C₆-Cycloalkyl, C₁-C₃-Haloalkyl, C₂-C₃-Haloalkenyl, C₂-C₃-Haloalkinyl, C₃-C₃-Halocycloalkyl, Halogen, Cyano, Nitro, C₁-C₃-Alkoxy, C₁-C₃-Haloalkoxy, C₁-C₃-Alkylthio, C₁-C₃-Alkylsulfinyl, C₁-C₃-Alkylsulfonyl, C₁-C₃-Haloalkylthio, C₁-C₃-Haloalkylsulfinyl, C₁-C₃-Haloalkylsulfonyl, C₁-C₃-Alkylamino, Di-(C₁-C₃-alkyl)amino und C₃-C₆-Cycloalkylamino
oder wobei die Substituenten unabhängig voneinander ausgewählt sein können aus Phenyl oder einem 5- oder 6-gliedrigen gesättigten oder ungesättigten heteroaromatischen Ring, wobei Phenyl oder der gesättigte oder ungesättigte heteroaromatische Ring gegebenenfalls einfach oder mehrfach, gleich oder verschieden mit C₁-C₃-Alkyl, C₁-C₃-Haloalkyl, Halogen, Cyano, Nitro, C₁-C₃-Alkoxy oder C₁-C₃-Haloalkoxy substituiert sein können,
- Q: steht ganz besonders bevorzugt für einen gegebenenfalls einfach oder mehrfach substituierten 5- oder 6- gliedrigen gesättigten oder ungesättigten heterocyclischen Ring der Reihe Q-7 bis Q-55, wobei die Substituenten unabhängig voneinander ausgewählt sein können aus Methoxy, Ethoxy, Fluor, Chlor , Brom, Jod, Methyl, Ethyl, Trifluormethyl, OCHF₂, OCF₃ , OCClF₂, SCH₃, SCF₃, SOCF₃, SO₂CF₃, Cyano und Nitro
- R⁵: steht bevorzugt für C₁-C₆-Alkyl, C₃-C₈-Cycloalkyl, C₁-C₄-Halogenalkyl, C₁-C₄-Alkoxy, C₁-C₄-Alkylthio, C₂-C₆-Alkenyl, C₂-C₆-Alkinyl, Aryl und Hetaryl steht, wobei diese Reste unsubstituiert sein können oder einen, zwei oder mehrere Reste aus der Gruppe C₁-C₆-Alkyl, C₃-C₆-Cycloalkyl, Halogen, Cyano, Nitro, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy und C₁-C₄-Halogenalkylthio, Aryl und Hetaryl tragen können, wobei die beiden letztgenannten Reste unsubstituiert sein können oder einen, zwei oder mehrere Reste aus der Gruppe Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können;
- R⁵: steht besonders bevorzugt für C₁-C₃-Alkyl, C₃-C₆-Cycloalkyl, C₁-C₃-Halogenalkyl, C₁-C₃-Alkoxy, C₁-C₃-Alkylthio, C₂-C₃-Alkenyl, C₂-C₃-Alkinyl, Aryl und Hetaryl steht, wobei Aryl und Hetaryl unsubstituiert sein kann oder einen, zwei oder mehrere Reste aus der Gruppe C₁-C₃-Alkyl, Halogen, Cyano, C₃-C₆-Cycloalkyl, C₁-C₃-Halogenalkyl, C₁-C₃-Alkoxy, C₁-C₃-Alkylthio, C₁-C₃-Alkylsulfinyl, C₁-C₃-Alkylsulfonyl, C₁-C₃-Halogenalkoxy und C₁-C₃-Halogenalkylthio tragen kann.
- R⁵: steht ganz besonders bevorzugt für Methyl, Ethyl, Cyclopropyl, Methoxy, Ethoxy, Trifluormethyl und Thiomethyl steht

Hervorgehoben sind Verbindungen der Formel (I) 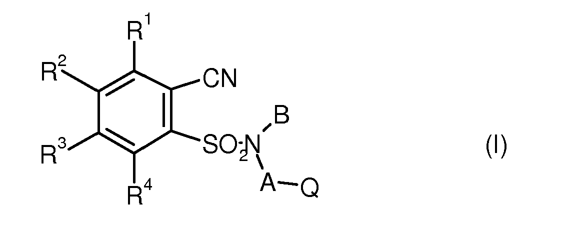 in welcher A, B, R¹, R², R³, R⁴ ,R⁵, Q und X die oben angegebenen allgemeinen, bevorzugten, besonders bevorzugten, ganz besonders bevorzugten Bedeutungen haben.

Weiterhin wurde gefunden, dass man Cyanobenzolsulfonsäureamide der Formel (I),
in welcher A, B, R¹, R², R³, R⁴, R⁵, Q und X
die oben angegebenen Bedeutungen haben, erhält, indem man
- (A): Heterocyclische Amine der Formel (II) 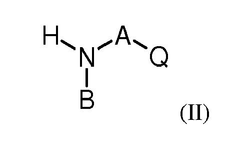
in welcher A, B und Q die oben angegebenen Bedeutungen haben,
mit Sulfonsäurechloriden der Formel (III) 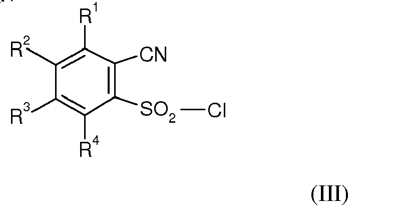 in welcher
- R¹,R², R³ und R⁴: die oben angegebenen Bedeutungen haben, in Gegenwart eines Säurebindemittels umsetzt,
- (B): Sulfonsäureamide der Formel (IV) 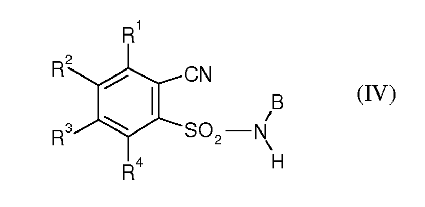 in welcher
B, R¹, R², R³ und R⁴ die oben angegebenen Bedeutungen haben, mit einer Verbindung der Formel (V)
in welcher

Q-A-Z (V)

- A und Q: die oben angegebenen Bedeutungen haben und Z Chlor, Brom , -O-SO₂-Alkyl oder -O-SO₂-Aryl sein kann, in Gegenwart eines Säurebindemittels umsetzt, oder indem man
- (C): Sulfonsäureamide der Formel (VI) 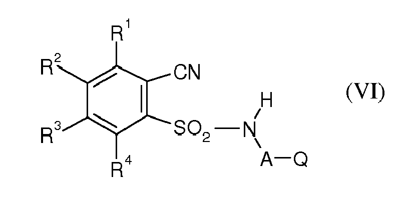 in welcher A, Q, R¹, R², R³ und R⁴ die oben angegebenen Bedeutungen haben, mit einer Verbindung der Formel (VII)

B-W (VII)
in welcher
- B: die oben angegebenen Bedeutungen hat und W Chlor, Brom, B-O-B, O-SO₂-Alkyl oder-O-SO₂-Aryl sein kann, in Gegenwart eines Säurebindemittels umsetzt.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

### Besonders günstige Mischpartner sind z.B. die folgenden:

### Fungizide:

Inhibitoren der Nucleinsäure Synthese
   Benalaxyl, Benalaxyl-M, Bupirimat, Chiralaxyl, Clozylacon, Dimethirimol, Ethirimol, Furalaxyl, Hymexazol, Mefenoxam, Metalaxyl, Metalaxyl-M, Ofurace, Oxadixyl, Oxolinsäure
Inhibitoren der Mitose und Zellteilung
   Benomyl, Carbendazim, Diethofencarb, Ethaboxam, Fuberidazole, Pencycuron, Thiabendazol, Thiophanat-methyl, Zoxamid
Inhibitoren der Atmungskette Komplex I
   Diflumetorim
Inhibitoren der Atmungskette Komplex II
   Boscalid, Carboxin, Fenfuram, Flutolanil, Furametpyr, Furmecyclox, Mepronil, Oxycarboxin, Penthiopyrad, Thifluzamid
Inhibitoren der Atmungskette Komplex III
   Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
   Entkoppler
   Dinocap, Fluazinam
Inhibitoren der ATP Produktion
   Fentinacetat, Fentinchlorid, Fentinhydroxid, Silthiofam
Inhibitoren der Aminosäure- und Proteinbiosynthese
   Andoprim, Blasticidin-S, Cyprodinil, Kasugamycin, Kasugamycinhydrochlorid Hydrat, Mepanipyrim, Pyrimethanil
Inhibitoren der Signal-Transduktion
   Fenpiclonil, Fludioxonil, Quinoxyfen
Inhibitoren der Fett- und Membran Synthese
   Chlozolinat, Iprodion, Procymidon, Vinclozolin
   Ampropylfos, Kalium-Ampropylfos, Edifenphos, Etridiazol, Iprobenfos (IBP), Isoprothiolan, Pyrazophos
   Tolclofos-methyl, Biphenyl
   Iodocarb, Propamocarb, Propamocarb hydrochlorid, Propamocarb-Fosetylat
Inhibitoren der Ergosterol Biosynthese
   Fenhexamid,
   Azaconazol, Bitertanol, Bromuconazol, Cyproconazol, Diclobutrazol, Difenoconazol, Diniconazol, Diniconazol-M, Epoxiconazol, Etaconazol, Fenarimol, Fenbuconazol, Fluquinconazol, Flurprimidol, Flusilazol, Flutriafol, Furconazol, Furconazol-cis, Hexaconazol, Imazalil, Imazalilsulfat Imibenconazol, Ipconazol, Metconazol, Myclobutanil, Nuarimol, Oxpoconazol, Paclobutrazol, Penconazol, Pefurazoat Prochloraz, Propiconazol, Prothioconazol, Pyrifenox, Simeconazol, Tebuconazol, Tetraconazol, Triadimefon, Triadimenol, Triflumizol Triforin, Triticonazol, Uniconazol, Voriconazol, Viniconazol,
   Aldimorph, Dodemorph, Dodemorphacetat, Fenpropidin, Fenpropimorph, Spiroxamin, Tridemorph,
   Naftifin, Pyributicarb, Terbinafin
Inhibitoren der Zellwand Synthese
   Benthiavalicarb, Bialaphos, Dimethomorph, Flumorph, Iprovalicarb, Mandipropamid, Polyoxins, Polyoxorim, Validamycin A
Inhibitoren der Melanin Biosynthese
   Carpropamid, Diclocymet, Fenoxanil, Phthalid, Pyroquilon, Tricyclazol
   Resistenzinduktion
   Acibenzolar-S-methyl, Probenazol, Tiadinil

### Multisite

Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram

### Weitere Fungizide

Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ferimzon, Flumetover, Flusulfamid, Fluopicolid, Fluoroimid, Fosetyl-Aluminium, Fosetyl-Calcium, Fosetyl-Natrium, Hexachlorobenzol, 8-Hydroxychinolinsulfat, Irumamycin, Isotianil, Methasulphocarb, Metrafenon, Methyl Isothiocyanat, Mildiomycin, Natamycin, Nickel dimethyldithiocarbamat, Nitrothalisopropyl, Octhilinon, Oxamocarb, Oxyfenthiin, Pentachlorphenol und Salze, 2-Phenylphenol und Salze, Piperalin, Propanosin-Natrium, Proquinazid, Pyribencarb, Pyrrolnitrin, Quintozen, Tecloftalam, Tecnazen, Triazoxid, Trichlamid, Valiphenal , Zarilamid,
2-(2-{[6-(3-Chlor-2-methylphenoxy)-5-fluorpyrimidin-4-yl]oxy}phenyl)-2-(methoxyimino)-N-methylacetamid,
2-[[[[1-[3(1Fluor-2-phenylethyl)oxy]phenyl]ethyliden]amino]oxy]methyl]-alpha-(methoxyimino)-N-methyl-alpha-benzacetamid,
cis-1-(4-Chlorphenyl)-2-(1H-1,2,4-triazol-1-yl)-cycloheptanol,
1-[(4-Methoxyphenoxy)methyl]-2,2-dimethylpropyl-1H-imidazol-1- carbonsäure,
2,3,5,6-Tetrachlor-4-(methylsulfonyl)-pyridin,
2-Butoxy-6-iod-3-propyl-benzopyranon-4-on,
2-Chlor-N-(2,3-dihydro-1,1,3-trimethyl-1H-inden-4-yl)-3-pyridincarboxamid,
3,4,5-Trichlor-2,6-pyridindicarbonitril,
3-[5-(4-Chlorphenyl)-2,3-dimethylisoxazolidin-3-yl]pyridin,
5-Chlor-7-(4-methylpiperidin-1-yl)-6-(2,4,6-trinuorophenyl)[1,2,4]triazolo[1,5-a]pyrimidin,
Methyl 2-[[[cyclopropyl[(4-methoxyphenyl) imino]methyl]thio]methyl]-.alpha.-(methoxymethylen)- benzacetat,
Methyl 1-(2,3-dihydro-2,2-dimethyl-1H-inden-1-yl)-1H-imidazole-5-carboxylat,
N-[2-(1,3-dimethylbutyl)phenyl]-5-fluor-1,3-dimethyl-1H-pyrazol-4-carboxamid,
N-(3',4'-dichlor-5-fluorbiphenyl-2-yl)-3-(difluormethyl)-1-methyl-1H-pyrazol-4-carboxamid,
N-(3-Ethyl-3,5,5-trimethyl-cyclohexyl)-3-formylamino-2-hydroxy-benzamid,
N-(4-Chlor-2-nitrophenyl)-N-ethyl-4-methyl-benzenesulfonamid,
N-(4-chlorbenzyl)-3-[3-methoxy-4-(prop-2-yn-1-yloxy)phenyl]propanamid,
N-[(4-chlorphenyl)(cyano)methyl]-3-[3-methoxy-4-(prop-2-yn-1-yloxy)phenyl]propanamid,
N-(5-Brom-3-chlorpyridin-2-yl)methyl-2,4-dichlornicotinamid,
N-[1-(5-Brom-3-chloropyridin-2-yl)ethyl]-2,4-dichloronicotinamid,
N-{(Z)-[(cyclopropylmethoxy) imino][6-(difluormethoxy)-2,3-difluorphenyl]methyl}-2-benzacetamid,
N-{2-[1,1'-bi(cyclopropyl)-2-yl]phenyl}-3-(difluormethyl)-1-methyl-1H-pyrazol-4-carboxamid,
N-{2-[3-Chlor-5-(trifluormethyl)pyridin-2-yl]ethyl}-2-(trifluoromethyl)benzamid,
N-ethyl-N-methyl-N'-{2-methyl-5-(trifluormethyl)-4-[3-(trimethylsilyl)propoxy]phenyl}imidoformamid,
N-ethyl-N-methyl-N'-{2-methyl-5-(difluormethyl)-4-[3-(trimethylsilyl)propoxy]phenyl}imidoformamid,
O-[1-[(4-Methoxyphenoxy)methyl]-2,2-dimethylpropyl]-1H-imidazol-1- carbothioic acid,
2-Amino-4-methyl-N-phenyl-5-thiazolcarboxamid,
2,4-Dihydro-5-methoxy-2-methyl-4-[[[[1-[3-(trifluoromethyl)-phenyl]-ethyliden]-amino]-oxy]-methyl]-phenyl]-3H-1,2,4-triazol-3-on (CAS Nr. 185336-79-2),
N-(6-Methoxy-3-pyridinyl)-cyclopropan carboxamid,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
   Carbamate,
      zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
   Organophosphate,
      zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
   Natrium-Kanal-Modulatoren / Spannungsabhängige Natrium-Kanal-Blocker
   Pyrethroide,
      zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R-isomer), Tralomethrin, Transfluthrin, ZXI 8901, Pyrethrins (pyrethrum)
   DDT
      Oxadiazine,
      zum Beispiel Indoxacarb
      Semicarbazone,
      zum Beispiel Metaflumizon (BAS320 I)
   Acetylcholin-Rezeptor-Agonisten/-Antagonisten
      Chloronicotinyle,
      zum Beispiel Acetamiprid, Clothianidin, Dinotefuran, Imidacloprid, Nitenpyram, Nithiazine, Thiacloprid, Thiamethoxam
      Nicotine, Bensultap, Cartap
   Acetylcholin-Rezeptor-Modulatoren
      Spinosyne,
      zum Beispiel Spinosad, Spinetoram
      GABA-gesteuerte Chlorid-Kanal-Antagonisten
      Organochlorine,
      zum Beispiel Camphechlor, Chlordane, Endosulfan, Gamma-HCH, HCH, Heptachlor, Lindane, Methoxychlor
      Fiprole,
      zum Beispiel Acetoprole, Ethiprole, Fipronil, Pyrafluprole, Pyriprole, Vaniliprole
   Chlorid-Kanal-Aktivatoren
      Mectine,
      zum Beispiel Abamectin, Emamectin, Emamectin-benzoate, Ivermectin, Lepimectin, Milbemycin
      Juvenilhormon-Mimetika,
      zum Beispiel Diofenolan, Epofenonane, Fenoxycarb, Hydroprene, Kinoprene, Methoprene, Pyriproxifen, Triprene
   Ecdysonagonisten/disruptoren
      Diacylhydrazine,
      zum Beispiel Chromafenozide, Halofenozide, Methoxyfenozide, Tebufenozide
      Inhibitoren der Chitinbiosynthese
      Benzoylharnstoffe,
      zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
      Buprofezin
      Cyromazine
   Inhibitoren der oxidativen Phosphorylierung, ATP-Disruptoren
      Diafenthiuron
      Organozinnverbindungen,
      zum Beispiel Azocyclotin, Cyhexatin, Fenbutatin-oxide
      Entkoppler der oxidativen Phosphorylierung durch Unterbrechung des H-Protongradienten
      Pyrrole,
      zum Beispiel Chlorfenapyr
      Dinitrophenole,
      zum Beispiel Binapacyrl, Dinobuton, Dinocap, DNOC, Meptyldinocap
      Site-I-Elektronentransportinhibitoren
      METI's,
      zum Beispiel Fenazaquin, Fenpyroximate, Flufenerim, Pyrimidifen, Pyridaben, Tebufenpyrad, Tolfenpyrad
      Hydramethylnon
      Dicofol
   Site-II-Elektronentransportinhibitoren
   Cyenopyrafen, Cyflumetofen, Rotenone
   Site-III-Elektronentransportinhibitoren
   Acequinocyl, Fluacrypyrim
   Mikrobielle Disruptoren der Insektendarmmembran
   Bacillus thuringiensis-Stämme
   Inhibitoren der Fettsynthese
      Tetronsäuren,
   zum Beispiel Spirodiclofen, Spiromesifen,
      Tetramsäuren,
   zum Beispiel Spirotetramat, cis-3-(2,5-dimethylphenyl)-4-hydroxy-8-methoxy-1-azaspiro[4.5]dec-3-en-2-on
      Oktopaminerge Agonisten,
   zum Beispiel Amitraz
   Inhibitoren der Magnesium-stimulierten ATPase,
   Propargite
      Nereistoxin-Analoge,
   zum Beispiel Thiocyclam hydrogen oxalate, Thiosultap-sodium
   Agonisten des Ryanodin-Rezeptors,
      Benzoesäuredicarboxamide,
   zum Beispiel Flubendiamid
      Anthranilamide,
   zum Beispiel Chlorantraniliprole (Rynaxypyr, 3-bromo-N-{4-chloro-2-methyl-6-[(methylamino)carbonyl]phenyl}1-(3-chloropyridin-2-yl)-1H-pyrazole-5-carboxamide)
   Biologika, Hormone oder Pheromone
   Azadirachtin, Bacillus spec., Beauveria spec., Codlemone, Metarrhizium spec., Paecilomyces spec., Thuringiensin, Verticillium spec.
   Wirkstoffe mit unbekannten oder nicht spezifischen Wirkmechanismen
      Begasungsmittel,
   zum Beispiel Aluminium phosphide, Methyl bromide, Sulfuryl fluoride
      Fraßhemmer,
   zum Beispiel Flonicamid, Pymetrozine, Pyrifluquinazone
      Milbenwachstumsinhibitoren,
   zum Beispiel Clofentezine, Diflovidiazin, Etoxazole, Hexythiazox
   Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Dicyclanil, Fenoxacrim, Fentrifanil, Flubenzimine, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Angießen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I behandelt werden. Die bei den Wirkstoffen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, ohne sie zu beschränken.

### Herstellungsbeispiele

### Beispiel 1

### 2-Cyano-N-methyl-N-(pyridin-2-ylmethyl)benzolsulfonamid (I-1)

Man legt 130 mg (1.06 mmol) N-Methyl-1-pyridin-2-ylmethanamin und 1 ml Triethylamin in 30 ml Tetrahydrofuran vor und versetzt mit 200 mg (0.99 mmol) 2-Cyano-benzolsulfonylchlorid. Es wird 3h bei Raumtemperatur und anschliessend 12h bei 50°C gerührt. Nach dem Abkühlen wird das Lösungsmittel i. Vak. entfernt. Danach erfolgt die Zugabe von Wasser, ges. Natriumhydrogencarbonat-Lösung und Essigester. Die wässrige Phase wird mehrfach mit Essigester extrahiert und die vereinigten organischen Phasen mit Magnesiumsulfat getrocknet und filtriert. Vom Filtrat wird das Lösungsmittel unter vermindertem Druck abdestilliert. Es verbleibt als Rückstand 0.25 g I-1 als grüner Feststoff.
¹H-NMR (400 MHz, DMSO, δ, ppm) der Verbindung (I-1): 2.88 (3 H), 4.52 (2 H), 7.26-7.36 (2 H), 7.56-7.60 (2 H), 7.76-7.92 (3 H), 8.05-8.10 (2 H), 8.43-8.44 (1 H).

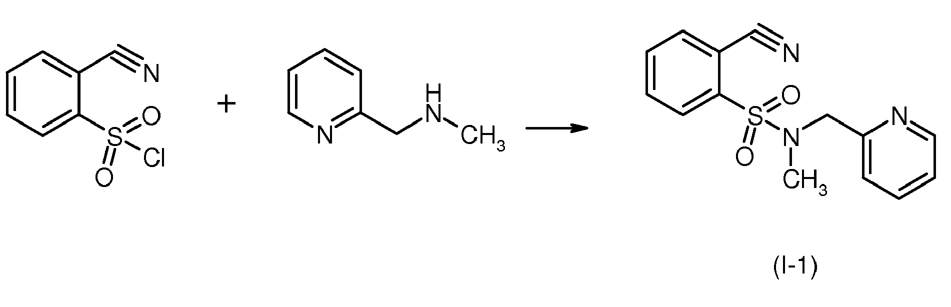

Analog zum oben aufgeführten Beispiel sowie der allgemeinen Beschreibung werden folgende Verbindungen der Formel (I) erhalten :

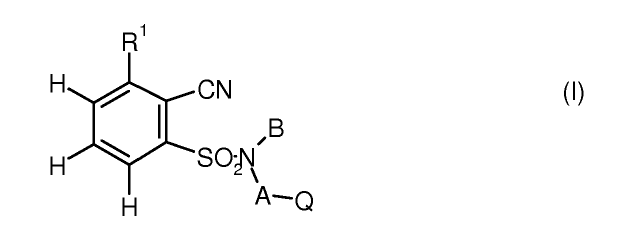

**Tabelle 1: Beispiele für die Verbindungen der Formel (I)**

| **Nr.** | **R¹** | **B** | **A** | **Q** |
|---|---|---|---|---|
| I-2 | F | CH₃ | CH₂ | 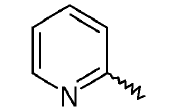 |
| I-3 | CH₃O | CH₃ | CH₂ | 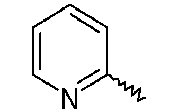 |
| I-4 | H | CH₃ | CH₂ | 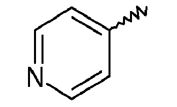 |
| I-5 | CH₃O | CH₃ | CH₂ | 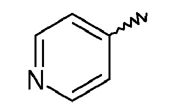 |
| I-6 | H | CH₃ | CH₂ | 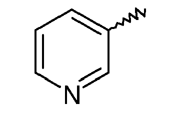 |
| I-7 | CH₃O | CH₃ | CH₂ | 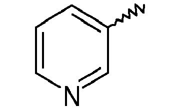 |
| I-8 | F | CH₃ | CH₂ | 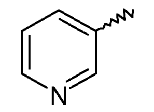 |
| I-9 | CH₃O | CH₃ | CH₂CH₂ | 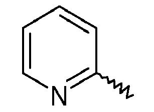 |
| I-10 | F | CH₃ | CH₂ | 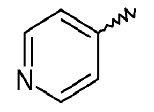 |
| I-11 | F | CH₂CH₃ | CH₂CH₂ | 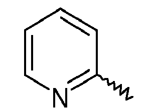 |
| I-12 | CH₃O | CH₂CH₃ | CH₂CH₂ | 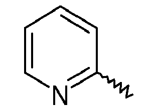 |
| I-13 | CH₃O | CH₂CH₂-CN | CH₂CH₂ | 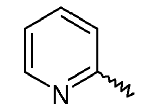 |
| I-14 | CH₂=CH-CH₂O | CH₃ | CH₂CH₂ | 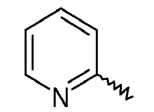 |
| I-15 | CH₃ | CH₃ | CH₂CH₂ | 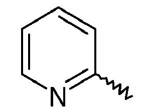 |
| I-16 | CH₃-O-CH₂CH₂O | CH₃ | CH₂CH₂ | 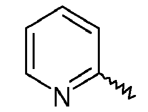 |
| I-17 | CH₃O | 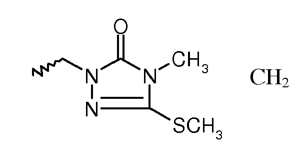 | | 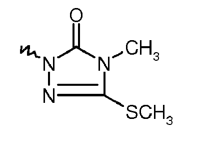 |
| I-18 | F | 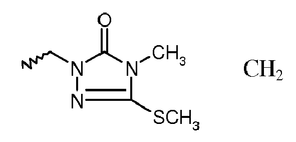 | | 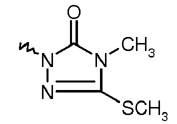 |
| I-19 | CH₃O | 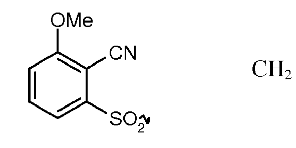 | | 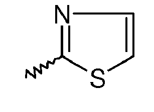 |
| I-20 | F | 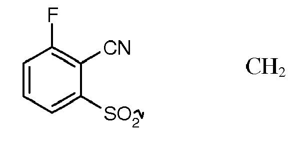 | | 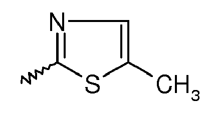 |
| I-21 | CH₃O | 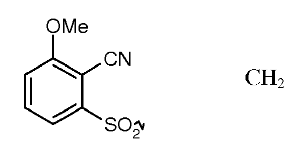 | | 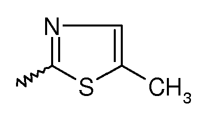 |
| I-22 | CH₃O | 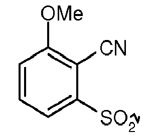 | CH₂ | 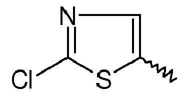 |
| I-23 | F | CH₃ | CH₂ | 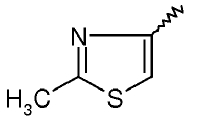 |
| I-20 | F | 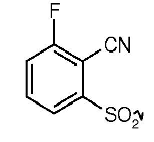 | CH₂ | 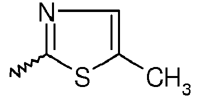 |
| I-21 | CH₃O | 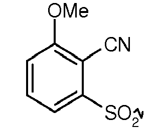 | CH₂ | 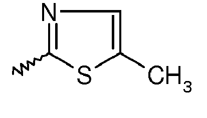 |
| I-22 | CH₃O | 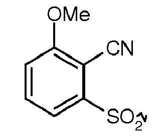 | CH₂ | 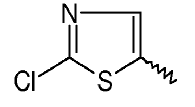 |
| I-23 | F | CH₃ | CH₂ | 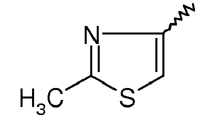 |
| I-24 | CH₃O | CH₃ | CH₂ | 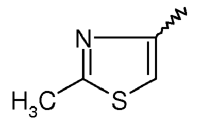 |
| I-20 | F | 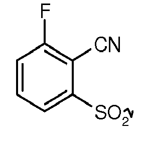 | CH₂ | 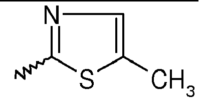 |
| I-21 | CH₃O | 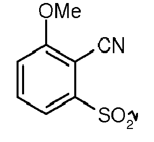 | CH₂ | 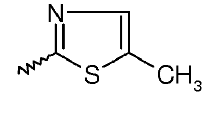 |
| I-22 | CH₃O | 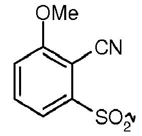 | CH₂ | 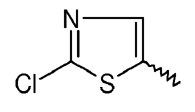 |
| I-23 | F | CH₃ | CH₂ | 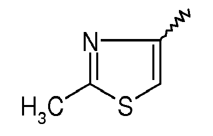 |
| I-24 | CH₃O | CH₃ | CH₂ | 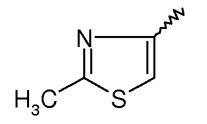 |
| I-25 | H | 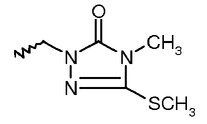 | CH₂ | 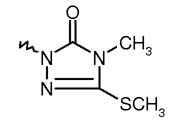 |
| I-26 | H | 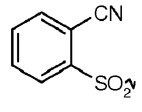 | CH₂ | 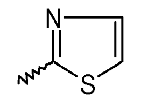 |
| I-27 | H | 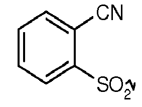 | CH₂ | 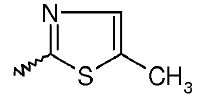 |
| I-28 | H | CH₃ | CH₂ | 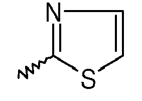 |
| I-29 | H | 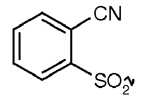 | CH₂ | 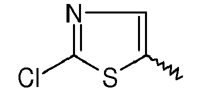 |

¹H-NMR-Daten:

Die Signale sind definiert durch eine chemische Verschiebung (in ppm) gegenüber Tetramethylsilan und ihr Integral, wobei die dazu korrespondierende Anzahl Wasserstoffatome jeweils in der Klammer angegeben ist.
I-2 (400 MHz, DMSO): 2.91 (3 H), 4.54 (2 H), 7.26-7.29 (1 H), 7.34-7.36 (1 H), 7.73-7.84 (2 H), 7.89-7.96 (2 H), 8.41-8.42 (1 H).
I-3 (400 MHz, DMSO): 2.86 (3 H), 4.01(3 H), 4.51 (2 H), 7.27-7.37 (2 H), 7.56-7.60 (2 H), 7.77-7.85 (2 H), 8.45-8.46 (1 H).
I-4 (400 MHz, DMSO): 2.80 (3 H), 4.45 (2 H), 7.29-7.31 (2 H), 7.87-8.15 (4 H), 8.55-8.56 (2 H).
I-5 (400 MHz, DMSO): 2.78 (3 H), 4.02 (3 H), 4.44 (2 H), 7.29-7.31 (2 H), 7.60-7.63 (2 H), 7.85-7.89 (1 H), 8.56 (2 H).
I-6 (400 MHz, DMSO): 2.77 (3 H), 4.44 (2 H), 7.37-7.40 (1 H), 7.70-7.73 (1 H), 7.86-7.97 (2 H), 8.08-8.14 (2 H), 8.51-8.53 (2 H).
I-7 (400 MHz, DMSO): 2.75 (3 H), 4.02 (3 H), 4.43 (2 H), 7.37-7.40 (1 H), 7.59-7.63 (2 H), 7.71-7.73 (1 H), 7.85-7.89 (1 H), 8.51 (2 H).
I-8 (400 MHz, DMSO): 2.80 (3 H), 4.45 (2 H), 7.38-7.41 (1 H), 7.59-7.63 (2 H), 7.72-7.74 (1 H), 7.83-8.04 (3 H), 8.52-8.53 (2 H).
I-9 (400 MHz, DMSO): 2.87 (3 H), 2.98 (2 H), 3.56 (2 H), 3.99 (3 H), 7.16-7.24 (2 H), 7.51-7.54 (2 H), 7.63-7.67 (1 H), 7.79-7.83 (1H), 8.40-8.41 (1 H).
I-10 (400 MHz, DMSO): 2.82 (3 H), 4.46 (2 H), 7.29-7.31 (2 H), 7.84-8.04 (3 H), 8.55-8.57 (2 H).
I-11 (400 MHz, DMSO): 1.08-1.12 (3 H), 2.96-2.99 (2 H), 3.40-3.45 (2 H), 3.64-3.68 (2 H), 7.15-7.22 (2 H), 7.62-7.96 (4 H), 8.38-8.39 (1 H).
I-12 (400 MHz, DMSO): 1.05-1.09 (3 H), 2.94-2.98 (2 H), 3.36-3.41 (2 H), 3.62-3.65 (2 H), 3.99 (3 H), 7.15-7.21 (2 H), 7.51-7.56 (2 H), 7.62-7.66 (1 H), 7.78-7.82 (1 H), 8.39-8.40 (1 H).
I-13 (400 MHz, DMSO): 2.80-2.84 (2 H), 2.95-2.99 (2 H), 3.67-3.70 (4 H), 3.99 (3 H), 7.14-7.19 (2 H), 7.53-7.65 (3 H), 7.79-7.83 (1 H), 8.36-8.37 (1 H).
I-14 (400 MHz, DMSO): 2.87 (3 H), 2.96-3.00 (2 H), 3.54-3.58 (2 H), 5.32-5.35 (1 H), 5.46-5.51 (1 H), 6.06 (1 H), 7.15-7.24 (2 H), 7.51-7.81 (4 H), 8.41-8.42 (1 H).
I-15 (400 MHz, DMSO): 2.55 (3 H), 2.88 (3 H), 2.96-3.00 (2 H), 3.55-3.58 (2 H), 7.15-7.23 (2 H), 7.63-7.83 (4 H), 8.38-8.40 (1 H).
I-16 (400 MHz, DMSO): 2.87 (3 H), 2.96-3.00 (2 H), 3.35 (3 H), 3.54-3.58 (2 H), 3.72-3.74 (2 H), 4.34-4.36 (2 H), 7.15-7.24 (2 H), 7.51-7.81 (4 H), 8.40-8.42 (1 H).
I-17 (400 MHz, CD3CN): 2.48 (6 H), 2.95 (6 H), 3.98 (3 H), 5.53 (4 H), 7.36 (1H), 7.53 (1 H), 7.66 (1 H).
I-18 (400 MHz, CD3CN): 2.46 (6 H), 2.97 (6 H), 5.52 (4 H), 7.57 (1 H), 7.77-7.80 (1 H), 7.85 (1 H).
I-19 (400 MHz, CD3CN): 4.00 (6 H), 5.68 (2 H), 7.42 (1H), 7.44 (2 H), 7.60 (1 H), 7.62 (2 H), 7.70 (2 H).
I-20 (400 MHz, CD3CN): 2.38 (3 H), 5.57 (2 H), 7.16 (1 H), 7.67 (2 H), 7.79-7.90 (2 H), 8.01 (2 H).
I-21 (400 MHz, CD3CN): 2.39 (3 H), 4.00 (6 H), 5.57 (1 H), 7.20 (1 H), 7.45 (2 H), 7.62 (2 H), 7.70 (2 H).
I-22 (400 MHz, CD3CN): 4.00 (6 H), 5.51 (2 H), 7.46 (2 H), 7.50 (2 H), 7.55 (1 H), 7.70 (2 H).
I-23 (400 MHz, CD3CN): 2.52 (3 H), 2.93 (3 H), 4.48 (2 H), 7.11 (1 H), 7.52-7.57 (1 H), 7.76-7.83 (2 H).
I-24 (400 MHz, CD3CN): 2.55 (3 H), 2.90 (3 H), 3.99 (3 H), 4.45 (2 H), 7.09 (1 H), 7.37 (1 H), 7.54 (1 H), 7.70 (1 H).
I-25 (400 MHz, CD3CN): 2.46 (6 H), 2.97 (6 H), 5.53 (4 H), 7.68-7.74 (2 H), 7.84-7.86 (1 H), 8.01-8.03 (1 H).
I-26 (400 MHz, CD3CN): 5.69 (2 H), 7.41 (1 H), 7.51 (1 H), 7.79-7.85 (4 H), 7.90-7.95 (2 H); 8.12-8.16 (2 H).
I-27 (400 MHz, CD3CN): 2.37 (3 H), 5.60 (2 H), 7.16 (1 H), 7.80-7.87 (4 H), 7.93-7.97 (2 H); 8.10-8.14 (2 H).
I-28 (400 MHz, CD3CN): 2.53 (3 H), 2.92 (3 H), 4.47 (2 H), 7.09 (1 H), 7.71-7.80 (2 H), 7.91 (1 H), 7.99 (1 H).
I-29 (400 MHz, CD3CN): 5.50 (2 H), 7.53 (1 H), 7.79-7.87 (4 H), 7.93 (2 H), 8.03 (2 H).

### Beispiele zur biologischen Wirksamkeit der erimdungsgemässen Verbindungen

### Beispiel Nr. 1

### Myzus-Test (MYZUPE Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis),* die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:

### Beispiel Nr. I-1, I-2, I-3, I-5, I-7, I-8, I-9, I-10, I-17

### Beispiel Nr. 2

### Tetranychus-Test, OP-resistent (TETRUR Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris*), die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 100 g/ha:

### Beispiel Nr. 1-5

### Beispiel Nr. 3

### Meloidogyne-Test (MELGIN Spritzbehandlung)

| | | |
|---|---|---|
| Lösungsmittel: | 80 | Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele eine Wirkung von ≥ 80 % bei einer Aufwandmenge von 20 ppm:
Beispiel Nr. I-1, I-2

## Patentansprüche

1. Verbindungen der Formel (I), 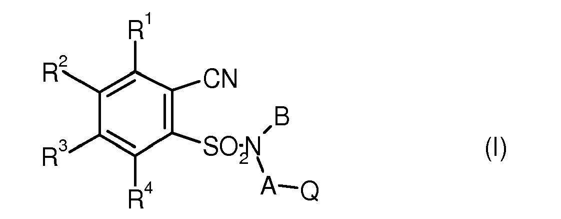 in welcher
A für C₁-C₆-Alkandiyl, C₃-C₆-Cycloalkyldiyl, C₂-C₆-Alkendiyl, C₁-C₆-Alkyl-C₂-C₆-Alkendiyl-, C₁-C₄-Alkyloxydiyl, C₁-C₄-Alkyloxy-C₁-C₄-alkyldiyl, C₁-C₄-Alkylthio-C₁-C₄-alkyldiyl, C₁-C₄-Alkylsulfinyl-C₁-C₄-alkyldiyl, C₁-C₄-Alkylsulfonyl-C₁-C₄-alkyldiyl steht, wobei diese Reste unsubstituiert sein können oder einen, zwei oder mehrere Reste aus der Gruppe Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino, Di-(C₁-C₄-alkyl)amino, Aryl oder Hetaryl tragen können;
B für A-Q, Cyano, C₁-C₆-Alkyl, C₃-C₈-Cycloalkyl, C₂-C₆-Alkenyl, C₁-C₆-Alkyl-C₂-C₆-Alkenyl, C₂-C₆-Alkinyl, C₁-C₄-Alkoxy, C₃-C₈-Cycloalkyl, -C(=X)-R⁵, C₁-C₄-Alkylsulfonyl, (C₁-C₄-Alkyl)amino, Di-(C₁-C₄-alkyl)amino, Aryl, Hetaryl, C₁-C₆-Arylalkyl und C₁-C₆-Hetarylalkyl steht, wobei die vier letztgenannten Reste unsubstituiert sein können oder einen, zwei oder mehrere Reste aus der Gruppe Halogen, Cyano, C₁-C₆-Alkyl, C₃-C₈-Cycloalkyl, Phenyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können;
B weiterhin für den Rest 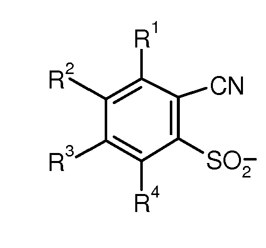 steht;
Q für einen 5-, 6- oder 7-gliedrigen gesättigten oder ungesättigten heterocyclischen Ring oder ein 9- oder 10-gliedriges annelliertes heterobicyclisches Ringsystem steht, wobei der Ring oder das Ringsystem, gegebenenfalls einfach oder mehrfach, gleich oder verschieden substituiert ist, und wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, Di-(C₁-C₄-alkyl)aminocarbonyl, (C₁-C₄-Alkyl)(C₁-C₄-Alkoxy)imino,
oder
wobei die Substituenten unabhängig voneinander ausgewählt sein können aus Phenyl oder einem 5- oder 6-gliedrigen heteroaromatischen Ring, wobei Phenyl oder der heteroaromatische Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy substituiert sein können;
R¹ für Wasserstoff, Halogen, C₁-C₆-Alkyl, C₁-C₆-Halogenalkyl, C₁-C₄-Alkoxy, C₃-C₆-Alkenyloxy, Methoxyethoxy oder C₁-C₄-Halogenalkoxy steht;
R², R³ und R⁴ unabhängig voneinander für Wasserstoff, Halogen, Cyano, Nitro, C₁-C₆-Alkyl, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy, C₁-C₄-Halogenalkylthio, C₂-C₆-Alkenyl, C₂-C₆-Alkinyl, C₁-C₄-Alkoxycarbonyl, Amino, (C₁-C₄-Alkyl)amino, Di-(C₁-C₄-alkyl)amino, Aminocarbonyl, (C₁-C₄-Alkyl)aminocarbony, Di-(C₁-C₄-alkyl)aminocarbonyl, Aryl und Hetaryl stehen, wobei die beiden letztgenannten Reste unsubstituiert sein können oder einen, zwei oder mehrere Reste aus der Gruppe Halogen, Cyano, C₁-C₆-Alkyl, C₃-C₈- Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können;
R⁵ für C₁-C₆-Alkyl, C₃-C₈-Cycloalkyl, C₁-C₄-Halogenalkyl, C₁-C₄-Alkoxy, C₁-C₄-Alkylthio, C₂-C₆-Alkenyl, C₂-C₆-Alkinyl, Aryl und Hetaryl steht, wobei diese Reste unsubstituiert sein können oder einen, zwei oder mehrere Reste aus der Gruppe C₁-C₆-Alkyl, C₃-C₆-Cycloalkyl, Halogen, Cyano, Nitro, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy und C₁-C₄-Halogenalkylthio, Aryl und Hetaryl tragen können, obei die beiden letztgenannten Reste unsubstituiert sein können oder einen, zwei oder mehrere Reste aus der Gruppe alogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können;
X für O oder S steht
sowie ihre N-Oxide und Salze.

2. Verbindungen der Formel (I) gemäß Anspruch 1, in welcher
A für C₁-C₆-Alkandiyl, C₃-C₆-Cycloalkyldiyl, C₂-C₆-Alkendiyl, C₁-C₆-Alkyl-C₂-C₆-Alkendiyl-, C₁-C₄-Alkyloxydiyl, C₁-C₄-Alkyloxy-C₁-C₄-alkyldiyl, C₁-C₄-Alkylthio-C₁-C₄-alkyldiyl, C₁-C₄-Alkylsulfinyl-C₁-C₄-alkyldiyl und C₁-C₄-Alkyl-sulfonyl-C₁-C₄-alkyldiyl steht, wobei diese Reste unsubstituiert sein können oder einen, zwei oder mehrere Reste aus der Gruppe Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino, Di-(C₁-C₄-alkyl)amino, Aryl oder Hetaryl tragen können;
B für A-Q, Cyano, C₁-C₆-Alkyl, C₃-C₈-Cycloalkyl, C₂-C₆-Alkenyl, C₁-C₆-Alkyl-C₂-C₆-Alkenyl, C₂-C₆-Alkinyl, C₁-C₄-Alkoxy, C₃-C₈-Cycloalkyl, -C(=X)-R⁵ , C₁-C₄-Alkylsulfonyl, (C₁-C₄-Alkyl)amino, Di-(C₁-C₄-alkyl)amino, Aryl, Hetaryl, C₁-C₆-Arylalkyl und C₁-C₆-Hetarylalkyl steht, wobei die vier letztgenannten Reste unsubstituiert sein können oder einen, zwei oder mehrere Reste aus der Gruppe Halogen, Cyano, C₁-C₆-Alkyl, C₃-C₈-Cycloalkyl, Phenyl, C₁-C₄-Alkoxy, C₁-C₄- Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können,
B weiterhin für 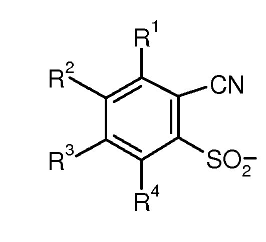 steht;
R¹ für Wasserstoff, Halogen, C₁-C₆-Alkyl, C₁-C₆-Halogenalkyl, C₁-C₄-Alkoxy steht;
R², R³ und R⁴ für Wasserstoff, Halogen, Cyano, Nitro, C₁-C₆-Alkyl, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy, C₁-C₄-Halogenalkylthio, C₂-C₆-Alkenyl, C₂-C₆-Alkinyl, C₁-C₄-Alkoxycarbonyl, (C₁-C₄-Alkyl)amino, Di-(C₁-C₄-alkyl)amino stehen;
Q für einen gegebenenfalls einfach oder mehrfach substituierten 5-, 6- oder 7-gliedrigen gesättigten oder ungesättigten heterocyclischen Ring oder ein 9- oder 10-gliedriges annelliertes heterobicyclisches Ringsystem der Reihe Q-1 bis Q-111 steht, wobei die Substituenten unabhängig voneinander ausgewählt sein können aus C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, Di-(C₁-C₄-alkyl)aminocarbonyl, (C₁-C₄-Alkyl)(C₁-C₄-Alkoxy)imino,
oder
wobei die Substituenten unabhängig voneinander ausgewählt sein können aus Phenyl oder einem 5- oder 6-gliedrigen heteroaromatischen Ring, wobei Phenyl oder der heteroaromatische Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy substituiert sein können, 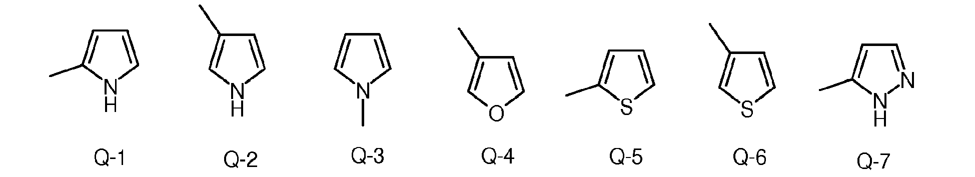 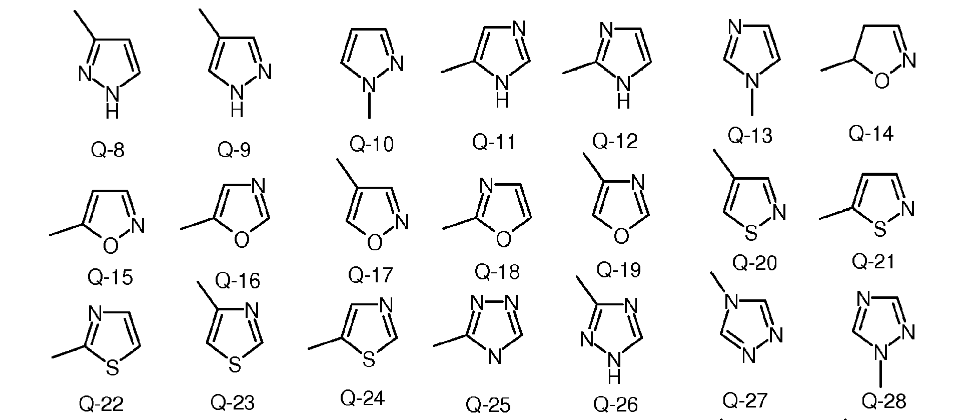 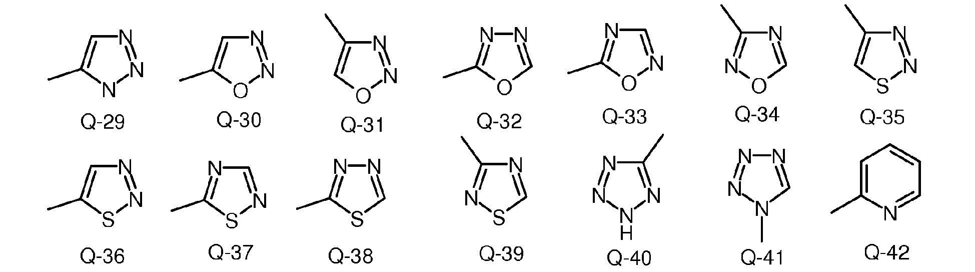 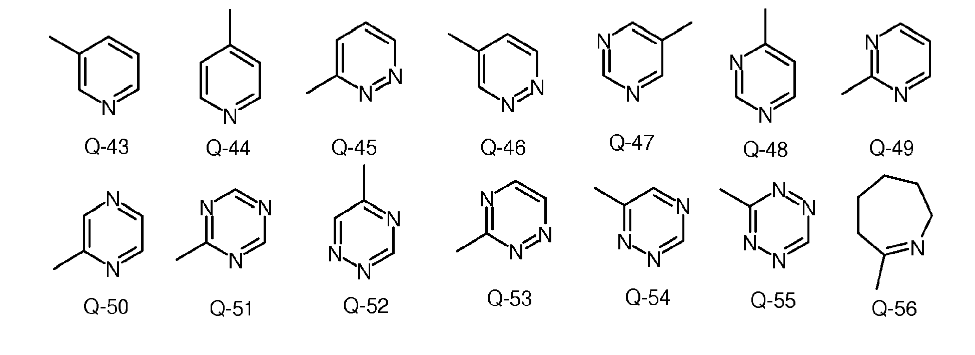 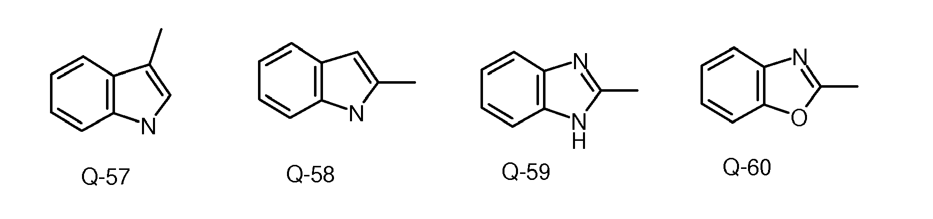 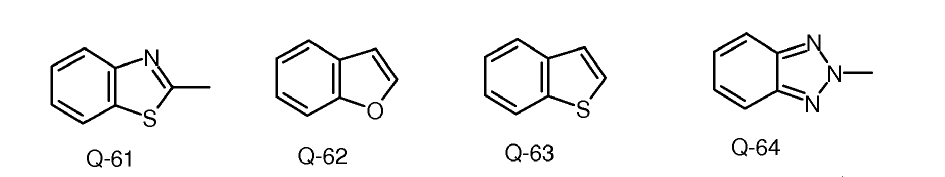 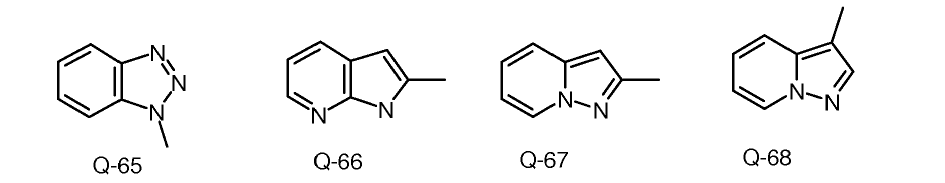 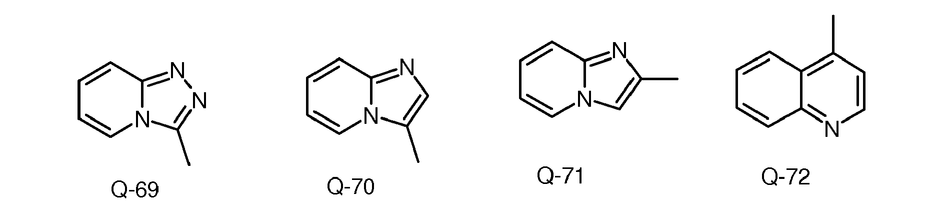 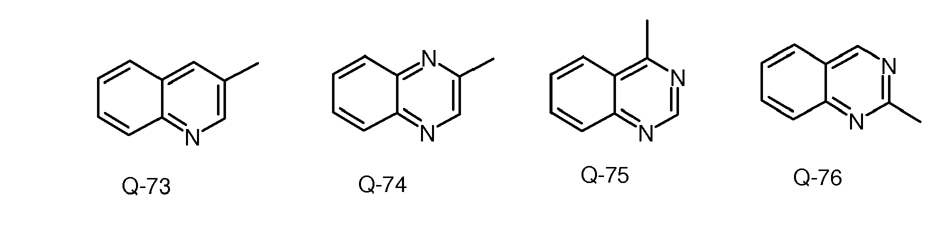 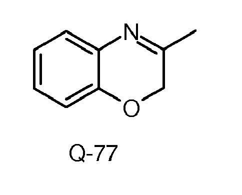 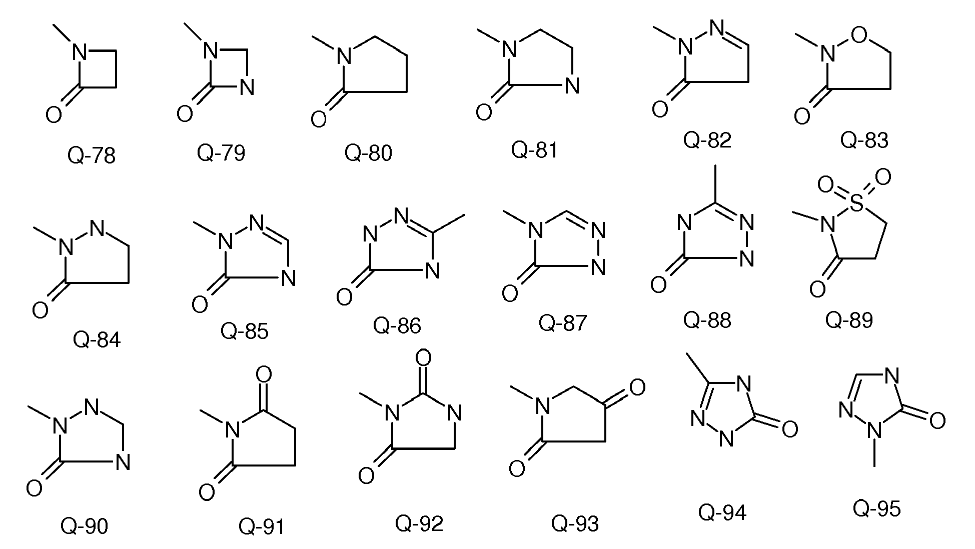 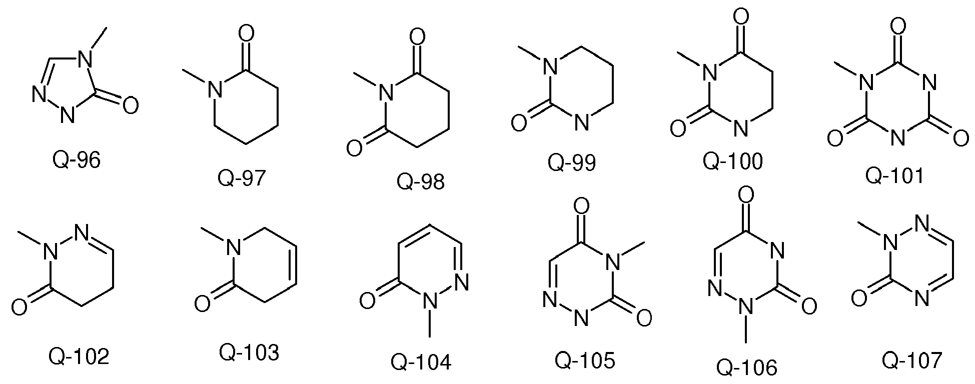 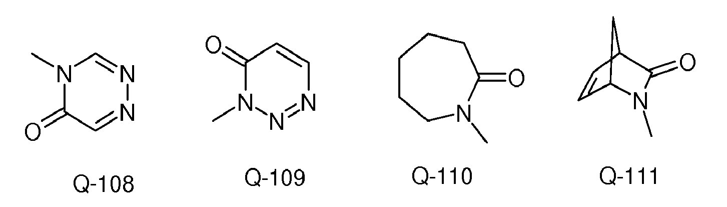
R⁵ für C₁-C₆-Alkyl, C₃-C₈-Cycloalkyl, C₁-C₄-Halogenalkyl, C₁-C₄-Alkoxy, C₁-C₄-Alkylthio, C₂-C₆-Alkenyl, C₂-C₆-Alkinyl, Aryl und Hetaryl steht, wobei diese Reste unsubstituiert sein können oder einen, zwei oder mehrere Reste aus der Gruppe C₁-C₆-Alkyl, C₃-C₆-Cycloalkyl, Halogen, Cyano, Nitro, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy und C₁-C₄-Halogenalkylthio, Aryl und Hetaryl tragen können, wobei die beiden letztgenannten Reste unsubstituiert sein können oder einen, zwei oder mehrere Reste aus der Gruppe Halogen, Cyano, C₁- C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino und Di-(C₁-C₄-alkyl)amino tragen können;

3. Verwendung von Verbindungen der Formel (I) gemäß den Ansprüchen 1 bis 2 zur Bekämpfung tierischer Schädlinge.

4. Verfahren zur Bekämpfung tierischer Schädlinge, **dadurch gekennzeichnet, dass** man Verbindungen der Formel (I) gemäß den Ansprüchen 1 bis 2 auf tierische Schädlinge und/oder phytopathogene Pilze und/oder deren Lebensraum und/oder Saatgut einwirken lässt.

5. Verfahren zur Herstellung agrochemischer Zusammensetzungen, **dadurch gekennzeichnet, dass** man Verbindungen der Formel (I) gemäß den Ansprüchen 1 bis 2 mit Streckmitteln und/oder oberflächenaktiven Stoffen vermischt.

6. Verwendung von Verbindungen der Formel (I) gemäß den Ansprüchen 1 bis 2 zur Behandlung von Saatgut.

7. Verwendung von Verbindungen der Formel (I) gemäß den Ansprüchen 1 bis 2 zur Behandlung von transgenen Pflanzen.

8. Verwendung von Verbindungen der Formel (I) gemäß den Ansprüchen 1 bis 2 zur Behandlung von Saatgut transgener Pflanzen.

9. Saatgut, welches mit einer Verbindung der Formel (I) gemäß den Ansprüchen 1 bis 2 behandelt wurde.
